# EUROPEAN PATENT SPECIFICATION

(11) **EP 1 224 155 B1**
(45) Date of publication and mention of the grant of the patent: **21.02.2007**
(21) Application number: 00984533.0
(22) Date of filing: 18.10.2000
(51) Int. Cl.: A61K 31/122, A61P 25/00, C07C 49/67, C07C 49/743, C07C 49/753

(54) **NOVEL BICYCLIC CANNABINOID AGONISTS FOR THE CANNABINOID RECEPTOR**
NEUE BIZYKLISCHE CANNABINOID-AGONISTEN FÜR DEN CANNABINOID-REZEPTOR
NOUVEAUX AGONISTES DE CANNABINOIDES BICYCLIQUES DESTINES AU RECEPTEUR DE CANNABINOIDES

(30) Priority: 18.10.1999 US 160239 P
(43) Date of publication of application: 24.07.2002
(73) Proprietor: The University of Connecticut, Farmington, CT 06032 (US)
(72) Inventor: MAKRIYANNIS, Alexandros, Willimantic, CT 06226 (US); KHANOLKAR, Atmaram, Coventry, RI 02816 (US)
(74) Representative: Beacham, Annabel Rose
(86) International application number: PCT/US2000/041238
(87) International publication number: WO 2001/028497

(56) References cited:
- US-A- 4 102 902
- US-A- 4 176 233
- US-A- 4 208 351
- US-A- 4 282 248
- US-A- 4 876 276
- US-A- 5 434 295
- US-A- 5 605 906
- US-A- 5 948 777
- BUSCH-PETERSEN ET AL: "Unsaturated Side Chain beta-11-Hydroxyhexahydrocannabinol Analogs" JOURNAL OF MEDICINAL CHEMISTRY, AMERICAN CHEMICAL SOCIETY. WASHINGTON, US, vol. 39, no. 19, 1996, pages 3790-3796, XP002109620 ISSN: 0022-2623
- HUFFMAN J W ET AL: "Stereoselective Synthesis of the Epimeric DELTA<7>-Tetrahydrocannabinols" TETRAHEDRON, ELSEVIER SCIENCE PUBLISHERS, AMSTERDAM, NL, vol. 51, no. 4, 23 January 1995 (1995-01-23), pages 1017-1032, XP004104971 ISSN: 0040-4020
- CHEMICAL ABSTRACTS, Columbus, Ohio, US; abstract no. 129:276026C, DRAKE ET AL.: 'Classical/nonclassical hybrid cannabinoids' XP002939621 & J. MED. CHEM. vol. 41, no. 19, 1998, pages 3596 - 3608
- HUFFMAN ET AL.: 'Synthesis of 5', 11-dihydroxy- 8-tetrahydrocannabinol' TETRAHEDRON vol. 53, no. 39, 1997, pages 13295 - 13306, XP002939619
- CHEMICAL ABSTRACTS, Columbus, Ohio, US; abstract no. 87:39223W, ARCHER ET AL.: 'Cannabinoids. 3. Synthetic approaches to 9-ketocannabinoids. Total synthesis of nabilone' XP002939620 & J. ORG. CHEM. vol. 42, no. 13, 1977, pages 2277 - 2284
- HUFFMAN ET AL.: 'Synthesis of both enantiomers of nabilone from a common intermediate. Enantiodivergent synthesis of cannabinoids' J. ORG. CHEM. vol. 56, no. 6, 1991, pages 2081 - 2086, XP002939618

## Description

The present invention relates generally to cannabinoid compounds and is more particularly concerned with new and improved cannabinoid compounds exhibiting high binding affinity and selectivity for the CB1 and CB2 cannabinoid receptors, pharmaceutical preparations employing these analogs and methods of administering therapeutically effective amounts of the preparations to provide a physiological effect.

### Background of the Invention

Classical cannabinoids such as the marijuana derived cannabinoid Δ⁹-tetrahydrocannabinol, (Δ⁹-THC) produce their pharmacological effects through interaction with specific cannabinoid receptors in the body. So far, two cannabinoid receptors have been characterized: CB1, a central receptor found in the mammalian brain and peripheral tissues and CB2, a peripheral receptor found only in the peripheral tissues. Compounds that are agonists or antagonists for one or both of these receptors have been shown to provide a variety of pharmacological effects. See, for example, Pertwee, R.G., Pharmacology of cannabinoid CB1 and CB2 receptors, Pharmacol. Ther., (1997) 74:129 - 180 and Di Marzo, V., Melck, D., Bisogno, T., DePetrocellis, L., Endocannabinoids: endogenous cannabinoid receptor ligands with neuromodulatory action, Trends Neurosci. (1998) 21:521 - 528.

There is considerable interest in developing cannabinoid analogs possessing high affinity for one of the CB1 or CB2 receptors. Such analogs may offer a rational therapeutic approach to a variety of disease states.

### Summary of the Invention

The inventive compounds have been found to act as agonists for the CB1 and CB2 receptors. The invention includes compounds selective for either the CB1 or CB2 receptors. Certain of the novel bicyclic cannabinoids possess surprisingly improved cannabinoid receptor affinity and/or specificity over known cannabinoids. Thus, one aspect of the invention is the novel cannabinoids represented by structural formula 1 and physiologically acceptable salts thereof. 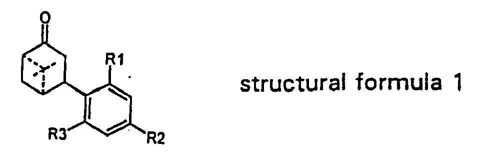 wherein R₁ is selected from the group consisting of OH; H; OCH₃; N₃; NH₂; O(CH₂)ₙN(CH₃)₂ and 1 - 3; 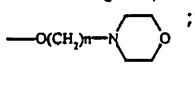 where n is an integer from
R₂ is selected from the group consisting of (CH₂)ₙCH₃, where n is an integer from 4 - 6; C(CH₃)₂(CH₂)ₙCH₃, where n is an integer from 3 - 5; 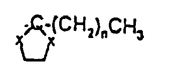 where X is selected from the group consisting of C, O, S and NH and n is an integer from 3 - 5; (CH₂)ₙC≡C where n is an integer from 3 - 5; C≡C(CH₂)ₙCH₃ where n is an integer from 2 - 4 and 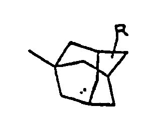 where R is (CH₂)ₙCH₃, where n is a maximum of 7;and
R₃ is selected from the group consisting of H; OH; OCH₃; N₃ and O(CH₂)ₙOH; where n is an integer from 1 - 5.
The novel cannabinoids are also more polar (less lipophilic) then known cannabinoids, which can improve their therapeutic usefulness in certain applications. Therefore, the novel compounds described herein, and physiologically acceptable salts thereof, represent potentially useful materials for providing a physiological effect to treat pain; peripheral pain; glaucoma; epilepsy; nausea such as associated with cancer chemotherapy; AIDS Wasting Syndrome; cancer; neurodegenerative diseases including Multiple Sclerosis, Parkinson's Disease, Huntington's Chorea and Alzheimer's Disease; to enhance appetite; to reduce fertility; to prevent or reduce diseases associated with motor function such as Tourette's syndrome; to prevent or reduce inflammation; to provide neuroprotection and to suppress memory and produce peripheral vasodilation. Thus, another aspect of the invention is the administration of a therapeutically effective amount of an inventive compound, or a physiologically acceptable salt thereof, to an individual or animal to provide a physiological effect.

### Description of Some Preferred Embodiments

As used herein a "therapeutically effective amount" of a compound, is the quantity of a compound which, when administered to an individual or animal, results in a sufficiently high level of that compound in the individual or animal to cause a discernible increase or decrease in stimulation of cannabinoid receptors. Physiological effects that result from cannabinoid receptor stimulation include analgesia, decreased nausea resulting from chemotherapy, sedation and increased appetite. Other physiological functions include relieving intraocular pressure in glaucoma patients and suppression of the immune system. Typically, a "therapeutically effective amount" of the compound ranges from about 10 mg/day to about 1,000 mg/day.

As used herein, an "individual" refers to a human. An "animal" refers to, for example, veterinary animals, such as dogs, cats, horses and the like, and farm animals, such as cows, pigs and the like.

The compound of the present invention can be administered by a variety of known methods, including orally, rectally, or by parenteral routes (e.g., intramuscular, intravenous, subcutaneous, nasal or topical). The form in which the compounds are administered will be determined by the route of administration. Such forms include, but are not limited to, capsular and tablet formulations (for oral and rectal administration), liquid formulations (for oral, intravenous, intramuscular or subcutaneous administration) and slow releasing microcarriers (for rectal, intramuscular or intravenous administration). The formulations can also contain a physiologically acceptable vehicle and optional adjuvants, flavorings, colorants and preservatives. Suitable physiologically to acceptable vehicles may include, for example, saline, sterile water, Ringer's solution, and isotonic sodium chloride solutions. The specific dosage level of active ingredient will depend upon a number of factors, including, for example, biological activity of the particular preparation, age, body weight, sex and general health of the individual being treated.

The novel cannabinoids can generally be described with reference to structural formula 1 and include physiologically acceptable salts thereof. 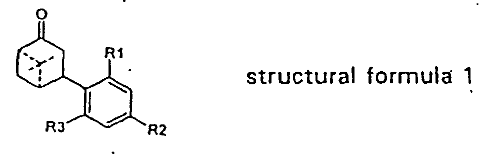 wherein R₁ is selected from the group consisting of OH; H; OCH₃; N₃; NH₂; O(CH₂)ₙN(CH₃)₂ and 1 - 3; 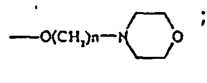 where n is an integer from
R₂ is selected from the group consisting of (CH₂)ₙCH₃, where n is an integer from 4-6; C(CH₃)₂(CH₂)ₙCH₃, where n is an integer from 3-5; 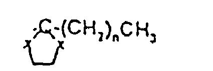 where X is selected from the group
consisting of C, O, S and NH and n is an integer from 3 - 5; (CH₂)ₙC≡C where n is an integer from 3 - 5; C≡C(CH₂)ₙCH₃ where n is an integer from 2 - 4 and 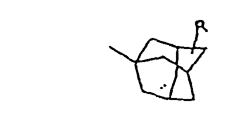 where R is (CH₂)ₙCH₃, where n is a maximum of 7;and
R₃ is selected from the group consisting of H; OH; OCH₃; N₃ and O(CH₂)ₙOH; where n is an integer from 1-5;
with the proviso that the compound of structural formula I is not:
(a) 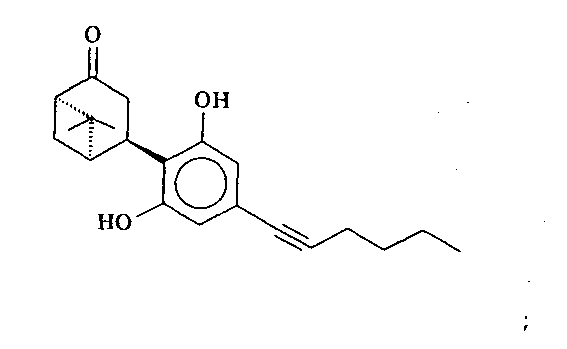
(b) 4-(2-methoxy-4-pentyl-6-hydroxyphenyl) -6,6-dimethylbicyclo[3.1.1]heptan-3-one;
(c) (-)-4-[2,6-dimethoxy-4-(1,1-dimethylheptyl)phenyl]-6,6-dimethylbicyclo[3.1.1]heptan-2-one;
(d) 4-[2-hydroxy-4-(1,1-dimethylheptyl)-6-methoxyphenyl]-6,6-methylbicyclo[3.1.1]heptan-2-one;
   or
(e) (+)-4-[4-(1,1-dimethylheptyl)-2,6-dihydroxyphenyl]-6,6-dimethyl-2-norpinanone.

The following examples are given for purposes of illustration only in order that the present invention may be more fully understood. These examples are not intended to limit in any way the practice of the invention. Material AM 1703 was prepared. Material AM 1703 can be represented by structural formula 1 when R₁ and R₃ are each OH and R₂ is 1,1-dimethylheptyl. Material AM 1703 is shown in structural formula 2.

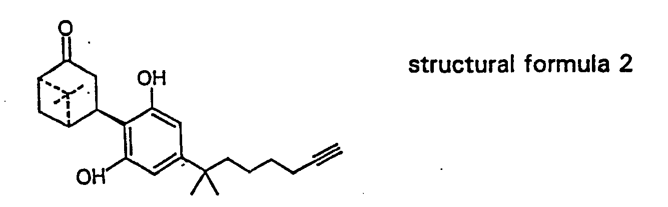

Material AM 1703 was prepared as follows.

### [7-(3,5-Dimethoxyphenyl-1,3-dithian-7-yl)-1-heptynyl]trimethysilane.

A solution of 5 g (19.5 mmol) of 2-(3,5-dimethoxyphenyl)-1,3-dithiane in 38 mL of dry tetrahydrofuran was cooled to -30 °C under argon and 14.5 mL of a 1.6 M solution (23.5 mmol) of n-butyllithium in hexanes was added dropwise. The yellow-brown reaction mixture was stirred at the same temperature for 2 hours (h) and 5.43 g (23.4 mmol, neat) of (6-bromo-1-hexynyl)trimethysilane was added in a dropwise manner when the color changed from yellow-brown to light yellow. The reaction mixture was allowed to warm to room temperature overnight and poured into water and extracted with diethyl ether. The combined organic extracts were dried and ether removed to afford the crude product which was purified on silica gel (15% diethyl ether-petroleum ether) to afford 6.81 g (86%) of the title compound as an oil. Anal. calcd. for C₂₁H₃₂O₂S₂Si C, 61.72; H, 7.89.

### [7-(3,5-Dimethoxyphenyl)-7-oxo-1-heptynyl]trimethylsilane.

A solution of 6.40 g (15.8 mmol) of [7-(3,5-dimethoxyphenyl-1,3-dithian-7-yl)-1-heptynyl]trimethysilane in 160 mL of 10% aqueous methanol was cooled in an ice-bath and 10.2 g (23.7 mmol, 1.5 equiv.) of bis(trifluoroacetoxy)iodobenzene was added portionwise with stirring. The reaction mixture was stirred for an additional 10 min and poured into 100 mL of sodium bicarbonate solution. The mixture was extracted with diethyl ether, ether extracts were combined, dried and ether removed to afford an oil which was chromatographed on silica gel to afford 4.5 g (90%) of the title compound. Anal. calcd. for C₁₈H₂₆O₃Si C, 67.88; H, 8.23.

### [7-(3,5-Dimethoxyphenyl)-7-methyl-1-octynyl]trimethysilane.

[7-(3,5-Dimetho-xyphenyl)-7-oxo-1-heptynyl]trimethysilane (1.50 g, 4.75 mmol) was dissolved in 10 mL of anhydrous ether, the solution was cooled in an ice-bath under argon and a 3.16 mL of a 3 M solution of methylmagnesium bromide (9.48 mmol) in ether was added dropwise. The light grey solution was allowed to warm to room temperature and stirred for an additional hour. The reaction mixture was poured into saturated ammonium chloride solution, the organic phase was separated and the aqueous phase was extracted with fresh diethyl ether. The combined ether extracts were dried and ether removed to afford 1.50 g (95%) of pure [7-(3,5-dimethoxyphenyl)-7-hydroxy-1-octynyl]trimethysilane as a viscous oil after passing through a short silica gel column.

The above tertiary carbinol (1.50 g, 4.52 mmol) was dissolved in 9 mL of anhydrous carbon tetrachloride and dry hydrogen chloride gas was bubbled through for 1 h. The solution was transferred to a separatory funnel with the aid of more carbon tetrachloride, washed with water and 10% sodium bicarbonate solution. The organic phase was dried and rotary evaporated to afford an oil which was passed through a short silica column to give 1.43 g (90%) of the pure [7-chloro-7-(3,5-dimethoxyphenyl)-1-octynyl]trimethysilane.

A solution of the above chloride (1.43 g, 4.08 mmol) in dry toluene was cooled to -30 °C under argon and 4.1 mL of a 2 M solution of trimethylaluminum in toluene was added in a slow dropwise manner. The resulting clear reaction mixture was stirred at room temperature for about 16 hours and then 5% aqueous hydrochloric acid was added in a very cautious manner. The organic layer was separated, washer with water, dried and toluene removed. The residual oil was chromatographed on silica gel to afford a colorless oil. Anal. calcd. for C₂₀H₃₂O₂Si C, 72.23; H, 9.70.

### 7-(3,5-Dimethoxyphenyl)-7-methyl-1-octyne (8-065).

[7-(3,5-Dimethoxyphenyl)-7-methyl-1-octynyl]trimethysilane (900 mg, 2.73 mmol) was dissolved in 3.5 mL of anhydrous methanol. Anhydrous potassium carbonate (75 mg, 0.55 mmol, 20 mol %) was added and the heterogeneous mixture was stirred at room temperature, under argon, for 24 h. The reaction mixture was diluted with water and extracted with diethyl ether. The ether extract was dried, concentrated by rotary evaporation and the residue was purified by chromatography on silica gel (5% ethyl ether-petroleum ether) to give 540 mg (76%) of the desilylated product. Anal. calcd. for C₁₇H₂₄O₂ C, 78.42; H, 9.29.

### 3-(1,1-Dimethylhept-6-ynyl)resorcinol (8-065).

A solution of 7-(3,5-dimethoxyphenyl)-7-methyl-1-octyne (445 mg, 1.71 mmol) in 17 mL of anhydrous dichloromethane was cooled to -40 °C under argon and 4.3 mL of a 1 M solution of boron tribromide (4.30 mmol) was added via syringe. The reaction mixture was allowed to warm to 0 °C with stirring over a period of 1 - 1.5 h and then quenched with saturated sodium bicarbonate. The organic layer was separated, dried and solvent removed. The residue was chromatographed on silica gel (30-40% ethyl ether-petroleum ether) to give 224 mg (56%) of the title resorcinol. Anal. calcd. for C₁₅H₂₀O₂ C, 77.55; H, 8.68.

**Coupling of 3-(1,1-dimethylhept-6-ynyl)resorcinol with nopinone diacetate.** A mixture of 224 mg (0.97 mmol) of 3-(1,1-dimethylhept-6-ynyl)resorcinol, 270 mg (0.97 mmol) nopinone diacetate and 185 mg (0.97 mmol) of p-toluenesulfonic acid monohydrate in 10 mL of chloroform was allowed to stand at room temperature for 4 h as described by Archer et al. After confirming the completion of the reaction by TLC, the reaction mixture was transferred to a separatory funnel and washed successively with 10% sodium bicarbonate, water, and dried. Solvent was removed and the residue was purified by flash chromatography on silica gel (30-40% ethyl ether- petroleum ether) to give 140 mg (40%) of the title bicyclic ketone (AM1703).

As used herein, "binding affinity" (Kᵢ) is represented by the IC₅₀ value which is the concentration of an analog required to occupy the 50% of the total number (Bmax) of the receptors. The lower the IC₅₀ value the higher the binding affinity. As used herein an analog is said to have "binding selectivity" if it has higher binding affinity for one receptor compared to the other receptor; e.g. a cannabinoid analog which has an IC₅₀ of 0.1 nanomoles (nM) for CB1 and 10 nM for CB2, is 100 times more selective for the CB1 receptor. The AM1703 material was tested for CB2 receptor binding affinity and for CB1 receptor affinity (to determine selectivity for the CB2 receptor).

For the CB1 receptor binding studies, membranes were prepared from rat forebrain membranes according to the procedure of P.R. Dodd et al, A Rapid Method for Preparing Synaptosomes: Comparison with Alternative Procedures, Brain Res., 107 - 118 (1981). The binding of the novel analogues to the CB1 cannabinoid receptor was assessed as described in W.A. Devane et al, Determination and Characterization of a Cannabinoid Receptor in a Rat Brain, Mol. Pharmacol., 34, 605 - 613 (1988) and A. Charalambous et al, 5'-azido Δ⁸ -THC: A Novel Photoaffinity Label for the Cannabinoid Receptor, J. Med. Chem., 35, 3076 - 3079 (1992) with the following changes. The above articles are incorporated by reference herein.

Membranes, previously frozen at -80 °C, were thawed on ice. To the stirred suspension was added three volumes of TME (25mM Tris-HCl buffer, 5 mM MgCl₂ and 1 mM EDTA) at a pH 7.4. The suspension was incubated at 4 °C for 30 min. At the end of the incubation, the membranes were pelleted and washed three times with TME.

The treated membranes were subsequently used in the binding assay described below. Approximately 30 µg of membranes were incubated in silanized 96-well microtiter plate with TME containing 0.1 % essentially fatty acid-free bovine serum albumin (BSA), 0.8 nM [³H] CP-55,940, and various concentrations of test materials at 30 °C for 1 hour. The samples were filtered using Packard Filtermate 196 and Whatman GF/C filterplates and washed with wash buffer (TME containing 0.5% BSA). Radioactivity was detected using MicroScint 20 scintillation cocktail added directly to the dried filterplates, and the filterplates were counted using a Packard Instruments Top-Count. Nonspecific binding was assessed using 100 nM CP-55,940. Data collected from three independent experiments performed with duplicate determinations was normalized between 100% and 0% specific binding for [³H] CP-55,940, determined using buffer and 100 nM CP-55,940. The normalized data was analyzed using a 4-parameter nonlinear logistic equation to yield IC₅₀ values. Data from at least two independent experiments performed in duplicate was used to calculate IC₅₀ values which were converted to Kᵢ values using the using the assumptions of Cheng et al, Relationship Between the Inhibition Constant (Kᵢ) and the concentration of Inhibitor which causes 50% Inhibition (IC₅₀) of an Enzymatic Reaction, Biochem. Pharmacol., 22, 3099-3102, (1973), which is incorporated by reference herein.

For the CB2 receptor binding studies, membranes were prepared from frozen mouse spleen essentially according to the procedure of P.R. Dodd et al, A Rapid Method for Preparing Synaptosomes: Comparison with Alternative Procedures, Brain Res., 226, 107 - 118 (1981) which is incorporated by reference herein. Silanized centrifuge tubes were used throughout to minimize receptor loss due to adsorption. The CB2 binding assay was conducted in the same manner as for the CB1 binding assay.

Binding affinities (Kᵢ) for both the CB1 and CB2 receptors are typically expressed in nanomoles (nM), although novel compound AM 1703 surprisingly exhibited a CB2 affinity of 0.59 picomoles (pM) and about a 500-fold CB2 selectivity over CB1. Other cannabinoid analogs have been reported that show some selectivity for the CB2 receptor. However the inventive analog described herein has surprisingly high affinity and selectivity for the CB2 receptor.

The physiological and therapeutic advantages of the inventive materials can be seen with additional reference to the following references, the disclosures of which are hereby incorporated by reference. Arnone M., Maruani J., Chaperon P, *et al,* Selective inhibition of sucrose and ethanol intake bv SR141716, an antagonist of central cannabinoid (CB1) receptors, Psychopharmacal, (1997) 132, 104-106. Colombo G, Agabio R, Diaz G. et al: Appetite suppression and weight loss after the cannabinoid antagonist SR141716. Life Sci. (1998) 63-PL13-PL117. Simiand J, Keane M, Keane PE, Soubrie P: SR 141716, A CB1 cannabinoid receptor antagonist, selectively reduces sweet food intake in marmoset. Behav. Pharmacol (1998) 9:179-181. Brotchie JM: Adjuncts to dopamine replacement a pragmatic approach to reducing the problem of dyskinesia in Parkinson's disease. Mov. Disord. (1998) 13:871-876. Terranova J-P, Storme J-J Lafon N et al: Improvement of memory in rodents by the selective CB1 cannabinoid receptor antagonist. SR 141716. Psycho-pharmacol (1996) 126:165-172. Hampson AL Grimaldi M. Axpirod J. Wink D: Cannabidiol and (-) Δ⁹ tetrahydrocannabinol are neuroprotective antioxidants. Proc. Natl Acad Sci. USA (1998) 9S:8268-8273. Buckley NE, McCoy Kl, Mpzey E et al Immunomodulation by cannabinoids is absent in mice deficient for the cannabinoid CB₂ receptor. Eur. J Pharmacol (2000) 396:141-149. Morgan Dr: Therapeutic Uses of Cannabis. Harwood Academic Publishers, Amsterdam. (1997). Joy JE, Wagtson SJ, Benson JA: Marijuana and Medicine Assessing the Science Base. National Academy Press, Washington, DC, USA (1999). Shen M. Thayer SA: Cannabinoid receptor agonists protect cultured rat hippocampal neurons from excitotoxicity. Mol. Pharmacol (1996) 54:459-462. DePetrocellis L, Melck D, Palmisano A. et al: The endogenous cannabinoid anandamide inhibits human breaast cancer cell proliferation. Proc Natl. Acad. Sci USA (1998) 95:8375-8380. Green K. Marijuana smoking vs. cannabinoids for glaucoma therapy. Arch. Ophibalmol. (1998) feb 433-1437. Hemming M, Yellowlees PM, Effective treatment of Tourette's syndrome with mariiuana. J. Psychopharmacol, (1993) 7:389-391. Muller-Vahl KB, Schneider U, Kolbe H, Emrich, HM. Treatment of Tourette's syndrome with delta-9-tatrahydrocannabinol. Am. J. Psychiat. (1999) 156-195. Muller-Vahl KB, Kolbe H, Schneider U, Emrich, HM Cannabis in movement disorders. Porsch. Kompicmentarmed (1999) 6 (suppl. 3) 23-27. Consroe P, Musty R, Rein J, Tillery W, Pertwee R. The perceived effects of smoked cannabis on Patents with multiple sclerosis, Eur. Neurol. (1997) 38-44-48. Pinnegan-Ling D, Musty R. Marinol and phantom limb pain: a case study. Proc Inv. Cannabinoid Rea. Sec. (1994):53. Brenneisen R, Pgli A, Elsohly MA, Henn V. Spiess Y: The effect of orally and rectally administered Δ⁹⁻ tetrahydrocannabinol on spasticity, a pilot study with 2 patients. Int. J. Clin Pharmacol Ther. (1996) 34:446-452. Martyn CN. Illis LS, Thom J. Nabilone in the treatment of multiple sclerosis. Lancet (1995) 345:579. Maurer M, Henn V, Dittrich A, Hofmann A. Delta-9-tetrahydrocannabinol shows antispastic and analgesic effects in a single case double-blind trial. Eur. Arch. Psychiat. Clin. Neurosci. (1990), Z40:1-4. Herzberg U, Eliav E, Bennett GJ, Kopin IJ: The analgesic effects of R(+) WIN 55,212-2 mesylate, a high affinity cannabinoid agonist in a rare model of neuropathic pain. Neurosci. Letts. (1997) 221:157-160. Richardson JD, Kilo S. Hargreaves KM, Cannabinoids reduce dryperalgesia and inflammation via interaction with peripheral CB1 receptors. Pain (1998) 75:111-119. Ricardson JD, Aanonsen I, Hargreaves KM: Antihyperalgesic effects of a spinal cannabinoids. Eur. J. Pharmacol. (1998) 346:145-153. Calignano A, La Rana G. Diuffrida A, Piomelli D: Control of pain initiation by endogenous cannabinoids. Nature (1998) 394:277-291. Wagner JA, Varga K, Jarai Z, Kunos G: Mesenteric vasodilation mediated by endothelia anandamide receptors. Hypertension (1999) 33:429-434. Schuel, H., Burkman, L.J., Picone, R.P., Bo, T., Makriyannis, A., Cannabinoid receptors in human sperm. Mol. Biol. Cell., (1997) (8), 325a.

The inventive analogs described herein, and physiologically acceptable salts thereof, have high potential when administered in therapeutically effective amounts for providing a physiological effect useful to treat pain; peripheral pain; glaucoma; epilepsy; nausea such as associated with cancer chemotherapy; AIDS Wasting Syndrome; cancer; neurodegenerative diseases including Multiple Sclerosis, Parkinson's Disease, Huntington's Chorea and Alzheimer's Disease; to enhance appetite; to reduce fertility; to prevent or reduce diseases associated with motor function such as Tourette's syndrome; to prevent or reduce inflammation; to provide neuroprotection and to suppress memory and produce peripheral vasodilation. Thus, another aspect of the invention is the administration of a therapeutically effective amount of an inventive compound, or a physiologically acceptable salt thereof, to an individual or animal to provide a physiological effect.

## Claims

1. A compound of the formula : 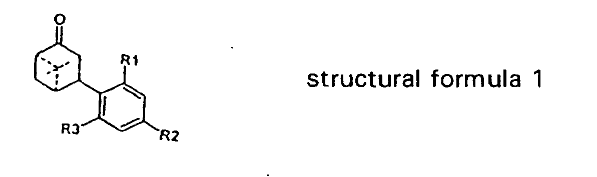 or a physiologically acceptable salt thereof,
wherein R₁ is selected from the group consisting of OH; H; OCH₃; N₃; NH₂; O(CH₂)ₙN(CH₃)₂ and 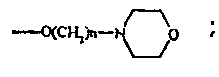 where n is an integer from 1 - 3;
R₂ is selected from the group consisting of (CH₂)ₙCH₃, where n is an integer from 4 - 6; C(CH₃)₂(CH₂)ₙCH₃, where n is an integer from 3-5; 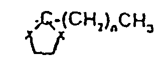 where each X is independently selected from the group
consisting of C, O, S and NH and n is an integer from 3 - 5; (CH₂)ₙC≡CH where n is an integer from 3 - 5; C≡C(CH₂)ₙCH₃ where n is an integer from 2 - 4 and 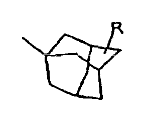 where R is (CH₂)ₙCH₃, where n is a maximum of 7; and
R₃ is selected from the group consisting of H; OH; OCH₃; N₃ and 0(CH₂)ₙOH; where n is an integer from 1- 5; for use in therapy.

2. The compound of claim 1 wherein R₁ and R₃ are each OH and R₂ is 1,1-dimethylheptyl, for use in therapy.

3. A compound as defined in claim 1 or claim 2, with the proviso that the compound of structural formula I is not:
(a) 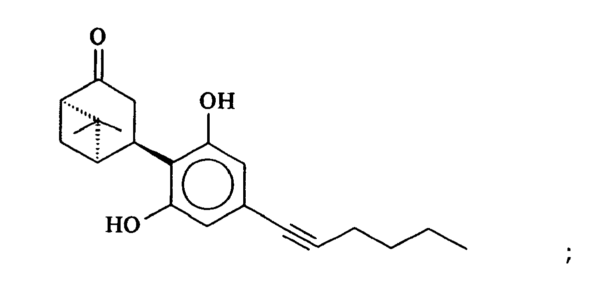
(b) 4-(2-methoxy-4-pentyl-6-hydroxyphenyl)-6,6-dimethylbicyclo[3.1.1]heptan-3-one;
(c) (-)-4-[2,6-dimethoxy-4-(1,1-dimethylheptyl)phenyl]-6,6-dimethylbicyclo[3.1.1]heptan-2-one;
(d) 4-[2-hydroxy-4-(1,1-dimethylheptyl)-6-methoxyphenyl]-6,6-methylbicyclo[3.1.1]heptan-2-one;
or
(e) (+)-4-[4-(1,1-dimethylheptyl)-2,6-dihydroxyphenyl]-6,6-dimethyl-2-norpinanone.

4. A compound as defined in claim 1 or claim 2, for use in preferentially stimulating the CB2 receptors in an individual or animal.

5. A pharmaceutical composition comprising a pharmaceutically acceptable carrier and a therapeutically effective amount of a compound as defined in claim 1 or claim 2.

## Patentansprüche

1. Eine Verbindung der Formel: 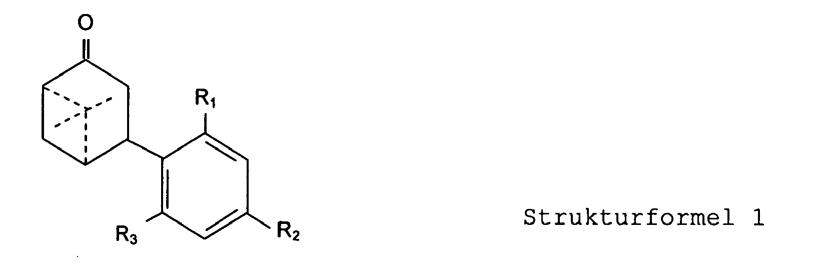 oder ein physiologisch annehmbares Salz davon,
wobei R₁ ausgewählt ist aus der Gruppe bestehend aus OH, H, OCH₃, N₃, NH₂, O(CH₂)ₙN(CH₃)₂ und 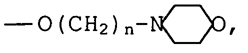 wobei n eine ganze Zahl von 1 bis 3 ist,
R₂ ausgewählt ist aus der Gruppe bestehend aus (CH₂)ₙCH₃, wobei n eine ganze Zahl von 4 bis 6 ist, C(CH₃)₂(CH₂)ₙCH₃, wobei n eine ganze Zahl von 3 bis 5 ist, 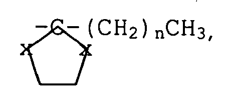 der wobei jedes X unabhängig ausgewählt ist aus
Gruppe bestehend aus C, O, S und NH und n eine ganze Zahl von 3 bis 5 ist,
(CH₂)ₙC≡CH, wobei n eine ganze Zahl von 3 bis 5 ist, C≡C(CH₂)ₙCH₃, wobei n eine ganze Zahl von 2 bis 4 ist und 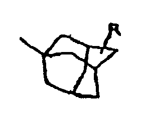 wobei R (CH₂)ₙCH₃ ist, wobei n ein Maximum von 7 ist, und
R₃ ausgewählt ist aus der Gruppe bestehend aus H, OH, OCH₃, N₃ und O(CH₂)ₙOH, wobei n eine ganze Zahl von 1 bis 5 ist, zur Anwendung bei einer Heilbehandlung.

2. Die Verbindung von Anspruch 1, wobei R₁ und R₃ jeweils OH und R₂ 1,1-dimethylheptyl ist, zur Verwendung bei einer Heilbehandlung.

3. Eine Verbindung nach Anspruch 1 oder 2, mit der Bedingung, dass die Verbindung der Strukturformel 1 nicht ist:
a) 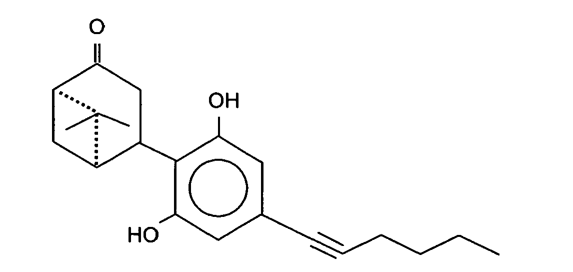
b) 4-(2-Methoxy-4-Pentyl-6-Hydroxyphenyl)-6, 6-Dimethylbicyclo[3.1.1]heptan-3-on,
c) (-)-4-[2,6-Dimethoxy-4-(1,1-Dimethylheptyl)phenyl]-6, 6-Dimethylbicyclo[3.1.1]heptan-2-on,
d) 4-[2-Hydroxy-4-(1,1-Dimethylheptyl)-6-Methoxyphenyl] -6,6-Methylbicyclo[3.1.1]heptan-2-on,
oder
e) (+)-4-[4-(1,1-Dimethylheptyl)-2,6-Dihydroxyphenyl]-6, 6-Dimethyl-2-Norpinanon.

4. Eine Verbindung nach Anspruch 1 oder 2 zur Verwendung zum vorzugsweisen Stimulieren der CB2 Rezeptoren in einem Individuum oder Tier.

5. Eine pharmazeutische Zusammensetzung mit einem pharmazeutisch annehmbaren Träger und einer therapeutisch wirksamen Menge einer Verbindung nach Anspruch 1 oder 2.

## Revendications

1. Composé de la formule : 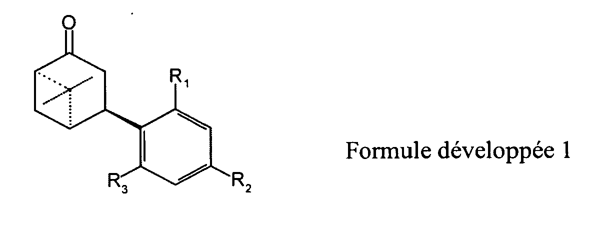 ou un sel physiologiquement acceptable de celui-ci,
dans lequel R₁ est choisi parmi le groupe constitué par OH; H; OCH₃; N₃; NH₂; O(CH₂)ₙN(CH₃)₂ et 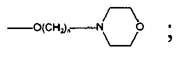 où n est un nombre entier allant de 1 à 3;
R₂ est choisi parmi le groupe constitué par (CH₂)ₙCH₃, où n est un nombre entier allant de 4 à 6; C(CH₃)₂(CH₂)ₙCH₃, où n est un nombre entier allant de 3 à 5; 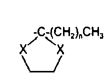 où chacun des X est indépendamment choisi parmi le groupe constitué par C, O, S et NH et n est un nombre entier allant de 3 à 5; (CH₂)ₙC≡CH où n est un nombre entier allant de 3 à 5; C≡C(CH₂)ₙCH₃ où n est un nombre entier allant de 2 à 4, et 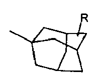 où R est (CH₂)ₙCH₃, où n est tout au plus égal à 7; et
R₃ est choisi parmi le groupe constitué par H; OH; OCH₃; N₃ et O(CH₂)ₙOH ; où n est un nombre entier allant de 1 à 5; pour une utilisation en thérapie.

2. Composé selon la revendication 1, dans lequel R₁ et R₃ sont chacun OH et R₂ est le 1,1-diméthylheptyle, pour une utilisation en thérapie.

3. Composé tel que défini dans la revendication 1 ou la revendication 2, sous réserve que le composé de formule développée 1 n'est pas :
(a) 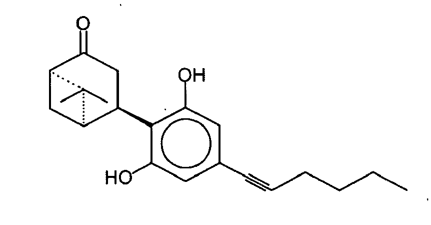
(b) 4-(2-méthoxy-4-pentyl-6-hydroxyphényl)-6,6 diméthyl-bicyclo [3.1.1]heptan-3-one;
(c) (-)-4-[2,6-diméthoxy-4-(1,1-diméthylheptyl)phényl]-6,6-diméthylbicyclo[3.1.1]heptan-2-one;
(d) 4-[2-hydroxy-4-(1,1-diméthylheptyl)-6-méthoxyphényl]-6,6-méthylbicyclo[3,1.1]heptan-2-one;
ou
(e) (+)-4-[4-(1,1-diméthylheptyl)-2,6-dihydroxyphényl]-6,6-diméthyl-2-norpinanone.

4. Composé tel que défini dans la revendication 1 ou la revendication 2, pour une utilisation dans la stimulation préférentielle des récepteurs CB2 chez un individu ou un animal.

5. Composition pharmaceutique comprenant un support pharmaceutiquement acceptable et une quantité thérapeutiquement efficace d'un composé tel que défini dans la revendication 1 ou la revendication 2.
